# EUROPEAN PATENT SPECIFICATION

(11) **EP 4 412 571 B1**
(45) Date of publication and mention of the grant of the patent: **21.01.2026**
(21) Application number: 22725902.5
(22) Date of filing: 02.05.2022
(51) Int. Cl.: A61F 13/511, A61F 13/512, A61F 13/15

(54) **ABSORBENT ARTICLE**
ABSORBIERENDER ARTIKEL
ARTICLE ABSORBANT

(30) Priority: 08.10.2021 WO PCT/EP2021/077900; 08.10.2021 WO PCT/EP2021/077906
(43) Date of publication of application: 14.08.2024
(73) Proprietor: Essity Hygiene and Health Aktiebolag, 405 03 Göteborg (SE)
(72) Inventor: BLOMSTRÖM, Philip, 405 03 GÖTEBORG (SE); PALMQVIST, Lisa, 405 03 GÖTEBORG (SE); KNÖS, Anna, 405 03 GÖTEBORG (SE)
(74) Representative: Hoffmann Eitle
(86) International application number: PCT/EP2022/061693
(87) International publication number: WO 2023/057096

(56) References cited:
- EP-A1- 0 983 758
- WO-A1-2018/047897
- WO-A1-2020/004648
- WO-A1-2020/112968
- WO-A1-2021/168513
- WO-A1-97/00057
- CN-A- 105 496 657
- GB-A- 2 262 906
- GB-A- 2 566 464
- US-A1- 2011 224 639
- US-B2- 10 888 469
- US-B2- 9 198 809

## Description

### TECHNICAL FIELD

The disclosure relates to a washable and reusable absorbent undergarment as well as a washable and reusable absorbent assembly.

### BACKGROUND

An absorbent article is worn for the purpose of absorbing body fluids such as urine and vaginal fluids. A washable and reusable absorbent undergarment comprises fabrics of woven or knitted materials. Conventionally such an absorbent undergarment comprises an integral absorbent assembly located in the crotch region of the wearer when the garment is worn. After use the absorbent garment is washed or laundered before reuse. The reusable absorbent articles have been mainly supplied for low and moderate volumes of body fluids. For sustainability reasons there has been an increased demand from users for reusable absorbent undergarments and consequently there is also a demand for such articles suitable for heavy flows of body fluids such as menstrual fluids. Menstrual fluids contain blood clots which tend to aggregate on the surface on the wearer facing top layer of the article.

GB 2566464, US 10,888,469 B2, EP 0 983 758 A1, WO 2018/047897 A1, US 9,198,809 B2, WO 2020/112968 A1, and CN 105496657 A form part of the state of the art relative to the present disclosure.

### SUMMARY

The present disclosure is based on the insight that there is a need for a washable and reusable absorbent undergarment and/or a reusable absorbent assembly able to handle viscous body fluids.

The present invention provides a washable and reusable absorbent undergarment according to claim 1 and/or a washable and reusable absorbent assembly according to claim 15.

The washable and reusable absorbent undergarment comprises an absorbent assembly and has an extension in the longitudinal direction and in the transversal direction. The absorbent assembly comprises a wearer facing top layer of a knitted material, an optional wicking layer beneath said top layer, a moisture barrier and at least one absorbent layer located between the top layer and the moisture barrier. The top layer comprises openings therein and the top layer has an open area of 25-95%.

The open area in the top layer may be 30-80%, such as 30-60%. Due to the large open area of the top layer, the absorbent assembly may receive high amounts of viscous fluid in a short time without giving the user a feeling of being overly wet. The openings will take care of blood clots that would otherwise stay on the surface and thus give an uncomfortable, wet feeling. The viscous fluid is directed to the layer beneath the top layer, i.e. to the optional wicking layer for further transport to the absorption layer(s), or to the absorption layer(s) for a direct and quick absorption and containment.

The openings may be arranged in rows. The rows may be transversal rows. The openings in adjacent rows may be offset in relation to each other. A "diamond" shaped macro pattern, i.e. the openings in a first row is offset with the openings in a second row adjacent the first row, such that each opening in the first row is situated centrally between two openings of the second row, may give better directional rewet properties, more even spreading and less wetting of each row than parallel rows of holes only. If one or more holes become clogged in one direction, there is also a higher risk of leakage due to "flooding" in that direction if the holes are in parallel rows compared with a diamond shaped hole pattern.

Another feature of the diamond or offset pattern is less sensitivity towards ripping in x and y directions leading to a higher durability than an even hole pattern due to fewer holes per length unit.

The openings may be non-circular. The openings may have an elongated shape. The openings may be elongated and have a ratio of length to width of >1, such as >1.5, and such as <10, such as <5. A non-circular opening, such as oval, triangular or bird's eye shaped openings may direct the flow of body liquids in different directions within the absorbent assembly.

The openings may have dimensions within the range of 0.5-4 mm, such as 0.5-2 mm, such as 1-3 mm. The openings should be large enough to take core of viscous fluids but still small enough not to collapse or expose the next layer of the absorbent assembly to the skin of the user. The distance between the openings may be 0.5-4 mm, such as 0.5-2 mm, such as 1-3 mm.

The top layer may be of a single layer. The top layer may not be a spacer fabric. The top layer may have a basis weight of 80-200 gsm. The top layer material may be of polyester, elastane or blends thereof.

The top layer may be selected from a warp knit, weft knit, jaquard knit, raschel knit and powernet.

A wicking layer may be located between the top layer and the absorbent layer. The basis weight of the wicking layer may be 180-250 gsm. The wicking layer may be of polyester, polyamide, elastane, or a mixture thereof. The wicking layer may be a jersey knit. The absorbent assembly may comprise two absorbent layers. The basis weight of an absorbent layer may be 200-350 gsm. The absorbent layer may be a hydrophilic French terry material and may be of polyester, polyamide, elastane, or a mixture thereof.

The absorbent undergarment may comprise a body fabric. The fabric may define a waist opening, two leg openings and a crotch region between the leg openings. The absorbent assembly may be permanently attached to the body fabric in a crotch region of the undergarment.

In a second aspect there is provided an absorbent assembly which may be used for and as a washable and reusable absorbent pad or napkin in a normal underwear. The features and advantages as set out in the above for the absorbent undergarment are equally applicable to the absorbent assembly for a pad.

### BRIEF DESCRIPTION OF THE DRAWINGS

The disclosure will be described in greater detail below with reference to the figures shown in the appended drawings, wherein
- Fig. 1: shows a top view of an absorbent undergarment with an absorbent assembly between the leg openings;
- Fig. 2: shows a close-up of a top layer material with openings;
- Fig. 3: shows a front view of an absorbent undergarment;
- Fig. 4: shows a cross-sectional view of a portion of an absorbent undergarment not including a wicking layer;
- Fig. 5: shows a cross-sectional view of a portion of an absorbent undergarment including a wicking layer.

### DETAILED DESCRIPTION

Different aspects of the present disclosure will be described more fully hereinafter with reference to the enclosed drawings. The embodiments of the absorbent undergarment disclosed herein can, however be realized in many different forms, such as different sizes and absorption levels, and should not be construed as being limited to the aspects set forth herein. In all figures in the following detailed description, the same reference numerals will be used to indicate the same elements.

As used herein, the term "absorbent undergarment" refers to garments that are worn and intended to be placed against the skin of the wearer to absorb and contain body fluids such as urine and vaginal fluids including menstrual fluid. The absorbent undergarment is an underwear of a fabric or textile for female use. The undergarment according to the present disclosure is intended to be laundered or otherwise restored after use for reuse as a sanitary article. The absorbent assembly may be used as and/or for a washable and reusable absorbent pad or napkin.

By "laundering" it is meant that the absorbent undergarment may be cleaned by laundering. The absorbent undergarment may thus be subjected to an aqueous solution containing detergent without losing its structural features. This aqueous solution may be heated as part of the laundering process, e.g. to 40°C or 60°C. The term "fabric" as used in the present disclosure may refer to single or multiple layers of fabrics. The fabric may be knitted or woven.

By "permanently attached", it is meant that the absorbent assembly is not intended to be separated from the body fabric before, during, or after use. The absorbent assembly as proposed herein is not necessarily directly bonded to the fabric, but instead may be attached via the leg opening seams or bonded by other means such as sealing tape. The absorbent assembly per se as proposed herein may or may not be permanently attached to an undergarment fabric material.

Knitting is a method of constructing a fabric by interlocking a series of loops of one or more yarns. There are two major classes of knitting namely warp and weft knitting.

Weaving is a method or process of interlacing two yarns of similar materials so that they cross each other at right angles to produce woven fabric. The warp yarns run lengthwise in the fabric and the filling threads (weft) or picks, run from side to side.

Jacquard is a system of weaving or knitting that utilizes a versatile pattern mechanism to produce intricate designs by using punch cards controlling the patterns produced. Jacquard knit may be valid for circular, flatbed, warp or weft knit.

Raschel knitting is a versatile type of warp knitting made in plain and jacquard patterns in which pillars of twisted yarns can create the mesh walls. The latter can be made with intricate eyelet and lacy patterns. Raschel fabrics are coarser than other warp-knit fabrics, but a wide range of fabrics can be made. Raschel knitting machines have one or two sets of latch needles and up to thirty sets of guides.

Powernet is a special type of elastic raschel knit or jacquard mesh with high strength, breathability and elasticity.

The top layer may be constructed of any suitable fabric, including naturally derived fibers selected from the group consisting of cotton, wool, silk, cellulose, regenerated cellulose, rayon, viscose, modal, lyocell, tencel, bamboo, hemp, flax, ramie, coir or banana. Alternatively, the top layer may be constructed of synthetic fibers selected from the group consisting of polyamide, acrylic, polyester or elastane, such as a mixture of polyester and elastane. Further, the top layer may be constructed of a blend or a mixture of naturally derived and/or synthetic fibers. The materials used for construction of the top layer should be soft and nonirritating to the skin and be readily penetrated by any body fluids. According to the present disclosure, the top layer should be launderable. The top layer may comprise between 20% and 100% naturally derived fibers, more preferably between 40% and 100% naturally derived fibers and most preferably between 60% and 100% naturally derived fibers. The top skin-facing layer comprises a water-permeable material thus allowing the body fluids to migrate to the underlying absorbent layer or layers. The top layer may have a basis weight of 80-200 gsm.

The top layer material has openings therein. The openings may be non-circular. The openings may have an elongated shape. The openings may be elongated and have a ratio of length to width of >1, such as >1.5, and such as <10, such as <5. A non-circular shape of the openings, such as oval, triangular or bird's eye shaped openings may direct the flow of body liquids in different directions within the absorbent assembly. The openings in the top layer may have dimensions within the range of 0.5-4.0, such as 1-2 mm.

A wicking layer may be provided underneath the top layer. The wicking layer has wicking features to allow the moisture to spread away from the wearer and into the absorbent layer or layers. Further, wicking enables an efficient spread of the moisture therefore allowing the moisture to be received in a wider area of the underlying one or more absorbent layers. This feature is particularly relevant for users suffering from stress incontinence as spurts of urine may cause the absorbent layer to be saturated rapidly at the point of impact. By spreading the volume of the fluid over a wider receiving area in the absorbent layer, the wicking features of the wicking layer increase the overall absorptive capacity of the absorbent layer. The wicking layer may be constructed of any suitable fabric, including naturally derived fibers or mixtures thereof selected from the group consisting of cotton, wool, silk, cellulose, regenerated cellulose, rayon, viscose, modal, lyocell, tencel, bamboo, hemp, flax, ramie, coir or banana. Alternatively, the wicking layer may be constructed of synthetic fibers or mixtures thereof selected from the group consisting of polyamide, acrylic, polyester or elastane. Further, the wicking layer may be constructed of a blend or a mixture of naturally derived and/or synthetic fibers. According to the present disclosure, the wicking layer should be launderable. The wicking layer may comprise between 20% and 100% naturally derived fibers, more preferably between 40% and 100% naturally derived fibers and most preferably between 60% and 100% naturally derived fibers. The basis weight of the wicking layer may be 180-250 gsm.

The absorbent assembly for use in an absorbent undergarment may contain one or multiple absorbent layers, capable of absorbing liquid and releasing the liquid during laundering. Release of the absorbed liquid may be beneficial as it enables the absorbent undergarment to be restored for reuse. Reusable absorbent undergarments provide a more sustainable alternative to the commonly used disposable hygiene articles that are not intended to be re-used. The absorbent layer may comprise any material capable of absorbing fluid, such as woven or nonwoven microfiber or polymer knits, fabric formed from hydrophilic fibers, absorbent fibers or powders. The absorbent layer may be of natural or synthetic fibers as described above for the other ingoing layers but may be of polyester and polyamide and contain odor treatment. The odor treatment may be silver ions, copper ions and zeolites or Polyhexamethylene biguanide, PHMB.

The absorbent layer may also comprise a material having an open cell porous structures such as high loft or synthetic fibers having reservoir properties. The absorbent layer may comprise between 20% and 100% naturally derived fibers, more preferably between 40% and 100% naturally derived fibers and most preferably between 60% and 100% naturally derived fibers. The basis weight of each absorbent layer may be 200-350 gsm. There may be one or more, such as 2, absorbent layers in the absorbent assembly.

The liquid-impermeable barrier layer may comprise any suitable material or combinations of material that prevents liquid from migrating from the absorbent assembly to the fabric layer. The liquid-impermeable barrier layer may comprise a hydrophobic woven or nonwoven material having inherent hydrophobic properties, or that has been treated to become hydrophobic. Examples of hydrophobic materials for treating the barrier layer are polymers such as silicone, polyurethane and combinations thereof. The liquid-impermeable barrier layer may comprise a microporous polymer film, e.g. a polyethylene, PTFE or polyurethane film, or combinations thereof. Laminates of polymer films and nonwoven materials may also be used. The liquid-impermeable barrier layer may be a coating of a moistureimpermeable material. The coating may be a polymer such as urethane wax on the surface facing away from the wearer. The liquid-impermeable barrier layer may be of polyurethane.

The liquid-impermeable barrier layer is preferably breathable, to allow vapor to escape from the absorbent undergarment, while preventing liquid from passing through the fabric layer. Further, the liquid-impermeable barrier layer may be constructed of an elastic material, thus providing the absorbent assembly with the required flexibility to adapt to the user's anatomy and movements. This improved fit increases the wearer's comfort during use and helps to prevent leakage from migrating through the undergarment's leg openings.

The absorbent undergarment comprises a body fabric. The fabric may define a waist opening, two leg openings and a crotch region between the leg openings. The absorbent assembly may be permanently attached to the body fabric. The body fabric of the undergarment may be constructed of any suitable fabric, including naturally derived fibers and mixtures thereof selected from the group consisting of cotton, wool, silk, cellulose, regenerated cellulose, rayon, viscose, modal, lyocell, tencel, bamboo, hemp, flax, ramie, coir or banana. Alternatively, the fabric may be constructed of synthetic fibers or mixtures thereof selected from the group consisting of polyamide, acrylic, polyester. Further, the fabric may be constructed of a blend or a mixture of naturally derived and/or synthetic fibers. The fibers may be recycled fibers. The fabric may comprise a stretchable fabric, e.g. elastane, so that the absorbent undergarment can provide a firm fit while at the same time adapting to the wearer's movements thus preventing any leakage from migrating through the leg openings and keeping the absorbent assembly in place. The fabric is preferably breathable to allow vapor to escape from the wearer's skin and from the absorbent structure.

The layers of the absorbent assembly and the undergarment body fabric may be bonded by conventional stitching, gluing techniques or via a bonding film such as tape.

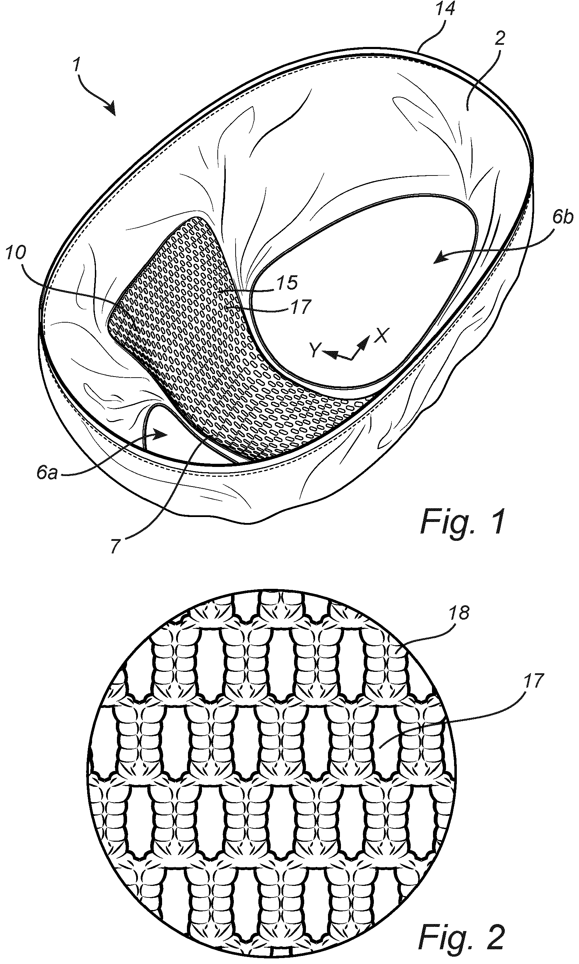

Fig. 1 discloses a washable and reusable absorbent undergarment 1 in the form of a panty comprising a fabric layer 2 defining a waist opening, two leg openings 6a, 6b and a crotch region 7 between the leg openings 6a, 6b. The absorbent undergarment 1 further comprises an absorbent assembly 10 covering at least part of the crotch region 7 and is permanently attached to the fabric layer 2. The absorbent assembly 10 comprises a knitted skin-facing top layer 15. The top layer 15 has openings 17 therein having a dimension of 0.5-4 mm. The top layer may be a single layer warp knitted material and may have a basis weight of 80-200 gsm. The openings 17 are arranged in transversal rows.

Figure 2 discloses a close-up of a single layer top layer 15 of a weft knit of the jacquard knit powernet type with threads 18 and oval openings 17. The openings 17 are arranged in rows, wherein the openings 17 in adjacent transversal rows are offset in relation to each other. The top layer 15 has an open area of 40% and the openings 17 therein being 1 mm wide and 2.5 mm long. The distance between the openings is 1 mm.

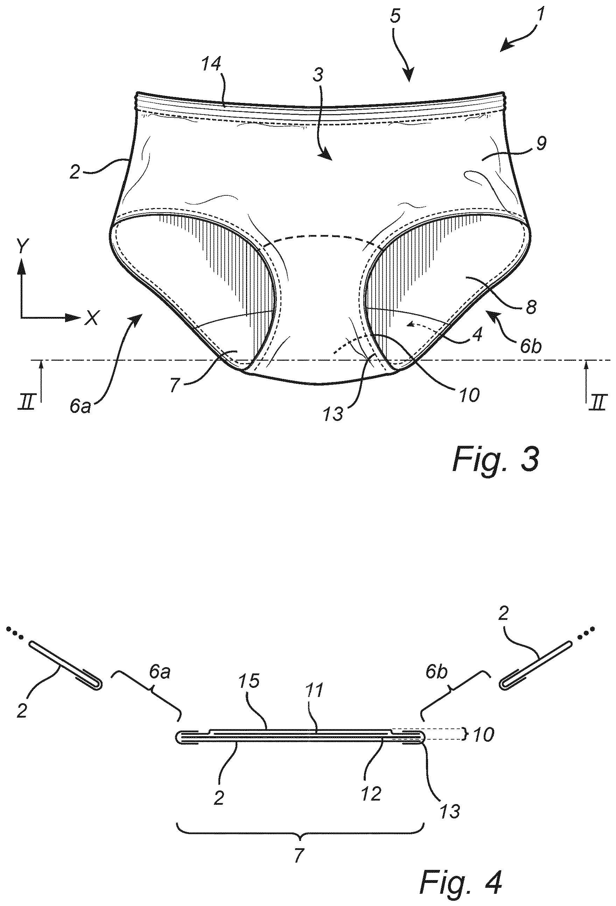

Figure 3 is a front view of an absorbent undergarment 1 comprising a fabric layer 2 having a front side 3, a back side 4 and a waist opening 5. The fabric layer 2 has two leg openings 6a and 6b, and a crotch region 7 in between the leg openings 6a and 6b. The fabric layer 2 further includes an interior surface 8, i.e. a surface of the fabric layer 2 that wholly or partially contacts the body of the wearer, and an exterior surface 9, i.e. a surface of the fabric layer 2 facing away from the wearer. The absorbent undergarment 1 further comprises an absorbent assembly 10 covering at least part of said crotch region 7. In figure 3, a leg opening seam 13 lines the leg openings 6a and 6b. The leg opening seam 13 may be provided by conventional stitching or gluing techniques. The leg opening seam 13 may comprise an elastic member such as elastic bonding film of polyurethane. Further, the leg opening seam 13 may serve to bond the absorbent assembly 10, such as the top layer 15, the absorbent layer(s) 11, the outer liquid impermeable barrier layer 12 and the wicking layer 16 together. The absorbent undergarment 1 may further comprise a waist band 14. The leg opening and the absorbent assembly may be sealed with a tape (not shown) to avoid side leakage at the leg openings.

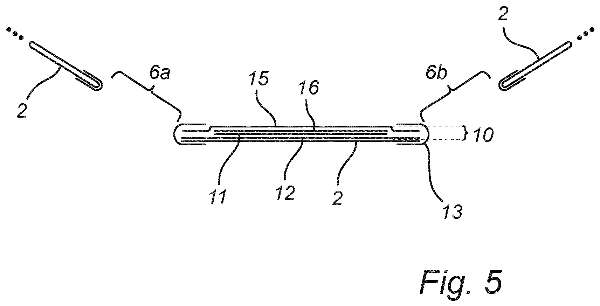

Figure 4 shows a cross-sectional view of the crotch region 7 of an absorbent undergarment 1. The absorbent undergarment 1 comprises the absorbent assembly 10, arranged on the interior surface 8 of the fabric layer 2 and covering a least part of the crotch region 7. The absorbent assembly 10 may comprise one or more absorbent layers 11. In figures 4 and 5, only one absorbent layer 11 is shown to increase clarity of the figures. Further, the absorbent assembly 10 comprises a liquid-impermeable barrier or barrier layer 12 located between the one or more absorbent layers 11 and the fabric layer 2. The liquid-impermeable barrier 12 prevents liquid from leaking through the one or more absorbent layers 11 into the fabric layer 2. The liquid-impermeable barrier 12 may be a coating on the wearer facing side of the fabric layer 2 or on a garment facing side of the absorbent layer 11. The absorbent assembly 10 further comprises a top layer 15 facing the skin of the user. The different layers of the absorbent assembly 10, but additionally also the fabric layer 2, are in Figure 4 bonded together via the leg opening seam 13.

Figure 5 shows an absorbent undergarment 1 wherein the absorbent assembly comprises a wicking layer 16 underneath the skin-facing top layer 15 and in contact with the absorbent layer 11. A liquid-impermeable barrier 12 is located between the absorbent layer 11 and the undergarment fabric 2.

### Methods

### Open area

Images of a knitted top layer material are taken using a modular high-performance optical stereomicroscope. A suitable instrument can be model MZ125 from Leica Microsystems, Germany, or equivalent. Representative samples are cut from top layer material and then analyzed. Many samples of the material are analyzed in case the top layer is heterogenous or varying and the respective open areas are averaged to reflect the whole top layer. The sample is laid flat on the reading surface of the microscope with the side of the material facing the user in the undergarment facing upwards. The background is selected to provide a good, sharp contrast. Wrinkles can be removed by gently stretching the sample but avoid overstretching which can distort sample geometry. The sample can be held in place by weights or tape along the sample periphery. Uniform light is provided from at least two sides of the sample to neutralize shadow effects and a suitable magnification and working distance is chosen so that the sample can be seen in clear focus. Using a high-quality digital camera (such as Digital Sight DS-Fi2 from Nikon, Japan, or equivalent) and relevant software (such as NIS Elements from Nikon, or equivalent), images with a rectangular field of view are acquired, suitably with pixel dimensions 2560 x 1920. An auto-focus step is performed and white balance is set. Exposure and gain are adjusted to obtain suitable contrast and detail between the sample and background.

Image analysis can be performed using software such as ImageJ (version 1.52p or above, available from National Institutes of Health, USA). A specimen image is opened in ImageJ. The image type is converted to 8 bit. The 8-bit grayscale image is then converted to a binary image with "black" foreground pixels corresponding to the opening regions using the "Minimum thresholding method". The thresholding method is ready-made in ImageJ but can otherwise be set according to this specification: If the histogram of gray level (GL) values (ranging from 0 to 255, one bin with propensity Pᵢ per gray level i) has exactly two local maxima, the threshold gray level value t is defined as that value for which Pₜ₋₁ > Pt and Pₜ≤Pₜ₊₁. If the histogram has greater than two local maxima, the histogram is iteratively smoothed using a windowed arithmetic mean of size 3, and this smoothing is performed iteratively until exactly two local maxima exist. The threshold gray level value t is defined as that value for which Pₜ₋₁ > Pt and Pₜ ≤ Pₜ₊₁. This procedure identifies the gray level (GL) value for the minimum population located between the dark pixel peak of openings and the lighter pixel peak of the material.

The open area percentage is calculated as the sum of the area of all openings (defined as above) in the image divided by the total area of the sample(s).

The dimension of the openings and the distance between the openings are measured with the above-mentioned equipment. The opening dimensions are measured from one side of the opening to the opposite side of the opening. The dimensions in all directions of an opening should be within the opening range mentioned herein, also in case of an irregular shape of the opening. In case the opening span more than 1 stitch, such as 2 stitches, i.e. more than 1 adjacent stitch, the opening dimension should be measured across the opening made up of the combined stitches, disregarding any possible crossing threads within the opening such as may be the case with tuck stitches. At least 10 openings and distances between openings should be measured as described above and an average of the measurements should be calculated.

The disclosure may be varied within the scope of the appended claims. For example, the materials and dimensions used for the different layers forming the absorbent insert may be varied, as indicated above.

## Claims

1. A washable and reusable absorbent undergarment (1) comprising an absorbent assembly (10), the absorbent undergarment (1) having an extension in the longitudinal direction (y) and in the transversal direction (x), the absorbent assembly (10) comprising a wearer facing top layer (15) of a knitted material, a moisture barrier (12) and at least one absorbent layer (11) located between the top layer (15) and the moisture barrier (12), wherein the top layer (15) comprises openings (17) therein and the top layer (15) has an open area of 25-95%, wherein the open area is determined by the method defined in the methods section herein.

2. Undergarment according to claim 1, wherein the top layer (15) has an open area of 30-80%, wherein the open area is determined by the method defined in the methods section herein.

3. Undergarment according to claim 1 or 2, wherein the top layer (15) has an open area of 30-60%, wherein the open area is determined by the method defined in the methods section herein.

4. Undergarment according any one of claims 1-3, wherein the top layer (15) is selected from a warp knit, weft knit, jaquard knit, raschel knit and powernet.

5. Undergarment according to any one of claims 1-4, wherein the top layer (15) is a powernet.

6. Undergarment according to any one of claims 1-5, wherein the openings (17) are elongated and has a ratio of length to width of >1.

7. Undergarment according to any one of claims 1-6, wherein the openings (17) are arranged in transversal (x) rows.

8. Undergarment according to any one of claims 1-7, wherein the openings are arranged in rows and adjacent rows are offset in relation to each other.

9. Undergarment according to any one of claims 1-8, wherein the top layer (15) is a single layer.

10. Undergarment according to any one of claims 1-9, wherein the top layer (15) has a basis weight of 80-200 gsm.

11. Undergarment according to any one of claims 1-10 wherein the absorbent assembly (10) comprises two absorbent layers (11).

12. Undergarment according to any one of claims 1-11, wherein the top layer (15) is not a spacer fabric.

13. Undergarment according to any one of claims 1-12, wherein the openings (17) have dimensions within the range of 0.5-4 mm

14. Undergarment according to any one of the claims 1-13, wherein the distance between the openings is 0.5-4 mm.

15. A washable and reusable absorbent assembly (10) having an extension in the longitudinal direction (y) and in the transversal direction (x), the absorbent assembly (10) comprising a wearer facing top layer (15) of a knitted material, an optional wicking layer (16) beneath said top layer (15), a moisture barrier (12) and at least one absorbent layer (11) located between the top layer (15) and the moisture barrier (12), wherein the top layer (15) comprises openings (17) therein and the top layer (15) has an open area of 25-95%, wherein the open area is determined by the method defined in the methods section herein.

## Patentansprüche

1. Waschbare und wiederverwendbare absorbierende Unterwäsche (1), umfassend eine absorbierende Anordnung (10), wobei die absorbierende Unterwäsche (1) eine Erstreckung in Längsrichtung (y) und in Querrichtung (x) aufweist, wobei die absorbierende Anordnung (10) eine trägerseitige Oberschicht (15) aus Maschenware, eine flüssigkeitsundurchlässige Barriere (12) und mindestens eine absorbierende Schicht (11) umfasst, die zwischen der Oberschicht (15) und der flüssigkeitsundurchlässigen Barriere (12) angeordnet ist, wobei die Oberschicht (15) Öffnungen (17) umfasst und die Oberschicht (15) einen offenen Flächenanteil von 25-95 % aufweist, wobei der offene Flächenanteil durch das im Verfahrensabschnitt definierte Verfahren bestimmt wird.

2. Unterwäsche nach Anspruch 1, wobei die Oberschicht (15) einen offenen Flächenanteil von 30-80 % aufweist, wobei der offene Flächenanteil durch das im Verfahrensabschnitt definierte Verfahren bestimmt wird.

3. Unterwäsche nach Anspruch 1 oder 2, wobei die Oberschicht (15) einen offenen Flächenanteil von 30-60 % aufweist, wobei der offene Flächenanteil durch das im Verfahrensabschnitt definierte Verfahren bestimmt wird.

4. Unterwäsche nach einem der Ansprüche 1-3, wobei die Oberschicht (15) ausgewählt ist aus Kettenwirkware, Schusswirkware, Jaquardwirkware, Raschelgewirk und Powernet.

5. Unterwäsche nach einem der Ansprüche 1-4, wobei die Oberschicht (15) ein Powernet ist.

6. Unterwäsche nach einem der Ansprüche 1-5, wobei die Öffnungen (17) länglich sind und ein Verhältnis von Länge zu Breite von > 1 aufweisen.

7. Unterwäsche nach einem der Ansprüche 1-6, wobei die Öffnungen (17) transversalen (x) Reihen angeordnet sind.

8. Unterwäsche nach einem der Ansprüche 1-7, wobei die Öffnungen in Reihen angeordnet sind und benachbarte Reihen zueinander versetzt sind.

9. Unterwäsche nach einem der Ansprüche 1-8, wobei die Oberschicht (15) einschichtig ist.

10. Unterwäsche nach einem der Ansprüche 1-9, wobei die Oberschicht (15) ein Flächengewicht von 80-200 g/m² aufweist.

11. Unterwäsche nach einem der Ansprüche 1-10, wobei die absorbierende Anordnung (10) zwei absorbierende Schichten (11) umfasst.

12. Unterwäsche nach einem der Ansprüche 1-11, wobei die Oberschicht (15) kein Abstandsgewebe ist.

13. Unterwäsche nach einem der Ansprüche 1-12, wobei die Öffnungen (17) Abmessungen im Bereich von 0,5-4 mm aufweisen

14. Unterwäsche nach einem der Ansprüche 1-13, wobei der Abstand zwischen den Öffnungen 0,5-4 mm beträgt.

15. Waschbare und wiederverwendbare absorbierende Anordnung (10), die eine Erstreckung in Längsrichtung (y) und in Querrichtung (x) aufweist, wobei die absorbierende Anordnung (10) eine trägerseitige Oberschicht (15) aus Maschenware, eine optionale feuchtigkeitsaufnehmende Schicht (16) unterhalb der Oberschicht (15), eine flüssigkeitsundurchlässige Barriere (12) und mindestens eine absorbierende Schicht (11) umfasst, die zwischen der Oberschicht (15) und der flüssigkeitsundurchlässigen Barriere (12) angeordnet ist, wobei die Oberschicht (15) Öffnungen (17) umfasst und die Oberschicht (15) einen offenen Flächenanteil von 25-95 % aufweist, wobei der offene Flächenanteil durch das im Verfahrensabschnitt definierte Verfahren bestimmt wird.

## Revendications

1. Un sous-vêtement absorbant lavable et réutilisable (1) comprenant un ensemble absorbant (10), le sous-vêtement absorbant (1) ayant une extension dans la direction longitudinale (y) et dans la direction transversale (x), l'ensemble absorbant (10) comprenant une couche supérieure (15) faisant face au porteur d'un matériau tricoté, un obstacle à l'humidité (12) et au moins une couche absorbante (11) située entre la couche supérieure (15) et l'obstacle à l'humidité (12), dans lequel la couche supérieure (15) comprend des ouvertures (17) dans celle-ci et la couche supérieure (15) a une aire ouverte de 25 à 95 %, où l'aire ouverte est déterminée par la méthode définie dans la section méthodes ici.

2. Sous-vêtement selon la revendication 1, dans lequel la couche supérieure (15) a une aire ouverte de 30 à 80 %, l'aire ouverte étant déterminée par la méthode définie dans la section méthodes ici.

3. Sous-vêtement selon la revendication 1 ou 2, dans lequel la couche supérieure (15) a une aire ouverte de 30 à 60 %, l'aire ouverte étant déterminée par la méthode définie dans la section méthodes ici.

4. Sous-vêtement selon l'une des revendications 1 à 3, dans lequel la couche supérieure (15) est choisie parmi un tricot de chaîne, un tricot de trame, un tricot Jaquard, un tricot Raschel et un powernet.

5. Sous-vêtements selon l'une des revendications 1 à 4, dans lequel la couche supérieure (15) est un powernet.

6. Sous-vêtement selon l'une des revendications 1 à 5, dans lequel les ouvertures (17) sont allongées et a un rapport longueur sur largeur de >1.

7. Sous-vêtement selon l'une des revendications 1 à 6, dans lequel les ouvertures (17) sont disposées en rangées transversales (x).

8. Sous-vêtement selon l'une des revendications 1 à 7, dans lequel les ouvertures sont disposées en rangées et les rangées adjacentes sont décalées les unes par rapport aux autres.

9. Sous-vêtement selon l'une des revendications 1 à 8, dans lequel la couche supérieure (15) est une seule couche.

10. Sous-vêtements selon l'une des revendications 1 à 9, dans lequel la couche supérieure (15) a un poids de base de 80 à 200 gsm.

11. Sous-vêtement selon l'une des revendications 1 à 10, dans lequel l'ensemble absorbant (10) comprend deux couches absorbantes (11).

12. Sous-vêtement selon l'une des revendications 1 à 11, dans lequel la couche supérieure (15) n'est pas un tissu entretoiseur.

13. Sous-vêtements selon l'une des revendications 1 à 12, dans lequel les ouvertures (17) ont des dimensions comprises entre 0,5 et 4 mm.

14. Sous-vêtement selon l'une des revendications 1 à 13, dans lequel la distance entre les ouvertures est de 0,5 à 4 mm.

15. Un ensemble absorbant lavable et réutilisable (10) ayant une extension dans la direction longitudinale (y) et dans la direction transversale (x), l'ensemble absorbant (10) comprenant une couche supérieure (15) faisant face au porteur d'un matériau tricoté, une couche d'évacuation (16) optionnelle sous ladite couche supérieure (15), un obstacle à l'humidité (12) et au moins une couche absorbante (11) située entre la couche supérieure (15) et l'obstacle à l'humidité (12), dans lequel la couche supérieure (15) comprend des ouvertures (17) dans celle-ci et la couche supérieure (15) a une aire ouverte de 25 à 95 %, où l'aire ouverte est déterminée par la méthode définie dans la section méthodes ici.
